# EUROPÄISCHE PATENTSCHRIFT

(11) **EP 0 580 894 B1**
(45) Veröffentlichungstag und Bekanntmachung des Hinweises auf die Patenterteilung: **14.01.1998**
(21) Anmeldenummer: 92113111.6
(22) Anmeldetag: 31.07.1992
(51) Int. Cl.: A61N 1/37

(54) **Vorrichtung zum Detektieren von Herzereignissen**
Device for detecting cardiac events
Dispositif de détection d'événements cardiaques

(43) Veröffentlichungstag der Anmeldung: 02.02.1994
(73) Patentinhaber: Pacesetter AB, 171 95 Solna (SE)
(72) Erfinder: Elmhammer, Agneta, S-115 24 Stockholm (SE); Dissing, Bo, S-164 75 Kista (SE)
(74) Vertreter: Lettström, Richard Wilhelm

(56) Entgegenhaltungen:
- EP-A- 0 321 764
- EP-A- 0 350 160
- WO-A-89/03705
- US-A- 4 708 144
- US-A- 4 768 511

## Beschreibung

Die Erfindung betrifft eine Vorrichtung zum Detektieren von Herzereignissen mit einem Signalaufnehmer zum Erfassen eines von den Herzereignissen abhängigen elektrischen Signals, einem dem Signalaufnehmer nachgeordneten Schwellenwertdetektor zum Erzeugen eines das Vorliegen eines Herzereignisses anzeigenden Detektionssignals, wenn das elektrische Signal einen vorgegebenen Schwellenwert überschreitet, mit einer Signalauswerteeinrichtung zur Bestimmung der aktuellen Signalhöhe des den Schwellenwert übersteigenden elektrischen Signals in bezug auf den Schwellenwert, mit einem der Signalauswerteeinrichtung nachgeordneten Mittelwertbildner zum Bilden eines Mittelwertes aus den während eines vorgegebenen Zeitintervalls bestimmten schwellenwertbezogenen Signalhöhen und mit einer Steuereinrichtung zum Einstellen des Schwellenwerts im Verhältnis zu dem erfaßten elektrischen Signal in Abhängigkeit von dem gebildeten Mittelwert, wobei der Schwellenwert im Verhältnis zu dem elektrischen Signal bei zunehmendem Mittelwert erhöht und bei abnehmendem Mittelwert verringert wird.

Eine derartige, aus der US-A-4 708 144 bekannte Vorrichtung ist Bestandteil eines implantierbaren Herzschrittmachers und dient dazu, über das elektrische Signal des intrakardialen Elektrogramms spontane Herzereignisse zu detektieren, um damit den Herzschrittmacher zu steuern. So ist es möglich, beispielsweise bei einem Ein-kammer-Herzschittmacher die Stimulierung des Herzens beim Auftreten von detektierten natürlichen Herzschlägen zu inhibieren oder bei einem Doppelkammer- Herzschrittmacher die Stimulation der einen Kammer mit den in der anderen Kammer detektierten Herzereignissen zu synchronisieren. Eine sichere Detektion von Herzeignissen erfordert eine Detektionsempfindlichkeit, die einerseits ausreichend hoch ist, um aus dem erfaßten elektrischen Signal die Herzaktivitäten detektieren zu können, andererseits aber nicht so hoch ist, daß Stör- und Rauschsignale fälschlicherweise als Hersereignisse detektiert werden. Ferner wird die Festlegung einer bestimmten Detektionsempfindlichkeit dadurch erschwert, daß sich das erfaßte elektrische Signal langfristig ändern kann, beispielsweise dadurch, daß die zur Ableitung des intrakardialen Elektrogramms verwendeten Elektroden nach ihrer Implantation über einen längeren Zeitraum im Herzen festwachsen. Bei der bekannten Vorrichtung erfolgt eine automatische Anpassung der Detektionsempfindlichkeit an das erfaßte elektrische Signal, indem Herzereignisse dadurch detektiert werden, daß das elektrische Signal einen Schwellenwert überschreitet; dabei wird der Schwellenwert bezogen auf das elektrische Signal in der Weise eingestellt, daß während eines Zeitintervalls mit einer Länge im Bereich von zumindest einigen Miuten bis in die Größenordnung von Stunden aus den Signalhöhen des den Schwellenwert bei jedem Herzereignis überschreitenden elektrischen Signals ein Mittelwert gebildet wird und daß das elektrische Signal, bevor es auf den Schwellenwertdetektor gelangt, einer in Abhängigkeit von dem Mittelwert veränderbaren Verstärkung unterzogen wird, während der Schwellenwert selbst auf einen unveränderlichen Wert festgesetzt ist.

Wie die US-A-4 827 934 zeigt, ist es aber auch möglich, den Schwellenwert selbst zur Anpassung der Detektionsempfindlichkeit an das elektrische Signal zu ändern. Da in diesem Fall der Schwellenwert veränderlich ist, wird als Parameter zum Einstellen des Schwellenwerts nicht die Signalhöhe selbst, sondern die Detektionsmarginale, also der jeweilige Abstand zwischen dem Schwellenwert und der Signalhöhe des den Schwellenwert überschreitenten elektrischen Signals herangezogen. Unter welchen Bedingungen im einzelnen eine Veränderung des Schwellenwertes erfolgt, geht jedoch aus der US-A-4 827 934 nicht hervor.

Bei der in der oben bereits genannten US-A-4 708 144 beschriebenen Anpassung der Detektionsempfindlichkeit wird davon ausgegangen daß sich die mit den zu detektierenden Herzereignissen korrelierenden Signalanteile des erfaßten elektrischen Signals nur sehr langsam ändern, weswegen die vorgenommene Mittelwertbildung über mehrere Minuten bis zu mehreren Stunden erfolgt. Dadurch gelingt es zwar, den Einfluß von Rauschsignalen auf die Einstellung der Detektionsempfindlichkeit fast völlig zu beseitigen, jedoch erfolgt die Anpassung der Detektionsempfindlichkeit beispielsweise an eine schnelle stetige oder eine plötzliche stufenförmige Veränderung des elektrischen Signals entsprechend langsam, da sich durch das lange Zeitintervall zur Mittellung der Signalhöhe des elektrischen Signals eine derartige Veränderung erst relativ spät in dem als Parameter zur Änderung der Detektionsempfindlichkeit dienenden Mittelwert bemerkbar macht. Derartige plötzliche oder schnelle Veränderungen des elektrischen Signals können auf plötzlich eintretenden Störbeeinflussungen wie Störsignalen von außen, Änderungen der Elektrodenposition, oder auf plötzlichen Änderungen der Herzsignale selbst, beispielsweise nach Extraschlägen des Herzens oder bei einem Infarkt, beruhen und können daher nicht von vorneherein ausgeschlossen werden.

Der Erfindung liegt die Aufgabe zugrunde, bei der Detektion von Herzereignissen eine automatische Anpassung der Detektionsempfindlichkeit an das vom Herzen abgenommenen elektrische Signal zu erreichen, wobei einerseits vereinzelt auftretende und rauschartige Änderungen des elektrischen Signals die Einstellung der Detektionsempfindlichkeit nicht beeinflussen, während andererseits schnelle substantielle Änderungen im elektrischen Signal zu einer angemessen schnellen Anpassung der Detektionsempfindlichkeit führen.

Gemäß der Erfindung wird die Aufgabe dadurch gelöst, daß bei der Vorrichtung der eingangs angegebenen Art die Länge des Zeitintervalls zur Mittelwertbildung der durchschnittlichen Dauer von einigen Atemzügen entspricht und daß die Steuereinrichtung Mittel zum Erzeugen einer Schalthysterese mit einem unteren und einem oberen Grenzwert aufweist, wobei eine Neueinstellung des Schwellenwerts jeweils nur dann erfolgt, wenn der Mittelwert entweder den unteren Grenzwert unterschreitet oder den oberen Grenzwert überschreitet.

Durch die Mittelwertbildung über das vergleichsweise kurze, weniger als eine Minute betragende Zeitintervall werden vereinzelt auftretende Störungen in dem elektrischen Signal, wie z.B. Extraschläge des Herzens, sowie atmungsbedingde Variationen des elektrischen Signals und Rauschsignale weitgehend unterdrückt. Darüber hinausgehende Beeinflussungen des Mittelwerts durch solche Störungen haben bei der erfindungsgemäßen Vorrichtung keinen Einfluß auf die Einstellung der Detektionsempfindlichkeit, weil die von den Störungen verursachten Änderungen des gebildeten Mittelwerts innerhalb der vorgesehenen Schalthysterese liegen. Auf der anderen Seite führen jedoch substantielle Änderungen des elektrischen Signals dazu, daß der Mittelwert auf Grund des nur kurzen Zeitintervalls für die Mittelwertbildung relativ schnell den oberen und unteren Grenzwert der Schalthysterese über- bzw. unterschreitet, so daß dementsprechend eine schnelle Anpassung der Detektionsempfindlichkeit an die Veränderungen in dem elektrischen Signal erfolgt. Auf diese Weise werden die gegensätzlichen Forderungen erfüllt, bei der automatischen Einstellung der Detektionsempfindlichkeit einerseits durch Mittelwertbildung den Einfluß von Störsignalen weitestmöglich zu unterdrücken und andererseits bei einer schnellen substantiellen Veränderung des erfaßten elektrischen Signals eine angemessen schnelle Anpassung der Detektionsempfindlichkeit zu erreichen. Schließlich besteht bei der erfindungsgemäßen Vorrichtung der Vorteil, daß wegen des nur kurzen Mittelungszeitintervalls der für die Mittelwertbildung erforderliche Rechenaufwand entsprechend gering ist.

Wie bereits im Zusammenhang mit dem eingangs genannten Stand der Technik angegeben, kann die Einstellung des Schwellenwerts im Verhältnis zu dem erfaßten elektrischen Signal, also die Änderung der Detektionsempfindlichkeit, entweder dadurch erfolgen, daß bei einem fest vorgegebenen Schwellenwert das dem Schellenwertdetektor zugeführte elektrische Signal einer varialblen Verstärkung unterliegt oder das der Schwellenwert selbst variiert wird. Im ersten Fall können die bei den detektierten Herzereignissen jeweils bestimmten Signalhöhen des elektrischen Signals direkt zur Mittelwertbildung herangezogen werden, während im zweiten Fall die Detektionsmarginale, also der Abstand zwischen den ermittelten Signalhöhen und dem jeweils aktuellen Schwellenwert der Mittelwertbildung unterliegt. Bei jeder Neueinstellung des Schwellenwerts ändern sich entsprechend die Werte für die zur Mittelwertbildung herangezogenen und auf den Schwellenwert bezogenen Signalhöhen. Um zu verhindern, daß dabei die vor der Schwellenwertänderung ermittelten und die Neueinstellung des Schwellenwerts verursacht habenden, auf den vorangegangenen Schwellenwert bezogenen Signalhöhen in den neuen Mittelwert eingehen, ist vorgesehen, daß nach jeder Neueinstellung des Schwellenwerts alle jeweils zur Bildung des Mittelwerts herangezogenen Signalhöhen auf den neuen Schwellenwert bezogen sind. Dies kann dadurch geschehen, daß die vor der Schwellenwertänderung ermittelten und in den neuen Mittelwert eingehenden Signalhöhen mit dem neuen Schwellenwert normiert werden oder das nach jeder Neueinstellung des Schwellenwerts nur die nach der Neueinstellung des Schwellenwerts ermittelten Signalhöhen zur Mittelwertbildung herangezogen werden.

Entsprechend einer vorteilhaften Weiterbildung der erfindungsgemäßen Vorrichtung ist die Länge des Zeitintervalls durch eine vorbestimmte Anzahl von vorzugsweise 10 bis 30 aufeinanderfolgend detektierten Herzereignissen vorgegeben. Beispielsweise können zur Mittelwertbildung die Signalhöhen der jeweils 18 zuletzt detektierten Herzereignissen herangezogen werden, was einer Mittelwertbildung über die Dauer von etwa 3 Atemzügen entspricht. Durch die Vorgabe der Anzahl der zur Mittelwertbildung herangezogenen Herzereignisse ist es in vorteilhafter Weise möglich, für die Mittelwertbildung einen Mittelwertspeicher mit einer entsprechenden begrenzten Anzahl von Speicherplätzen vorzusehen, wobei bei jedem detektierten Herzereignis der Wert der dabei ermittelten Signalhöhe des elektrischen Signals anstelle des jeweils ältesten abgespeicherten Werts in den Speicher eingelesen wird.

Die erfindungsgemäße Vorrichtung kann Bestandteil eines invertierenden Herzschrittmachers sein, bei dem die Abgabe eines Stimulationsimpulses dann erfolgt, wenn nicht innerhalb eines auf eine vorangegangene Stimulation oder Detektion eines spontanen Herzereignisses folgenden Basiszeitintervalls ein spontanes Herzereignis durch die erfindungsgemäße Vorrichtung detektiert wird. Um nach einem Stimulationsimpuls oder einer Folge von Stimulationsimpulsen das Auftreten eines natürlichen Herzereignisses sicher detektieren zu können, wird in vorteilhafter Weise nach einer auf eine Detektion eines spontanen Herzereignisses erstmalig folgenden Abgabe eines Stimulationsimpulses der Schwellenwert des Schwellenwertdetektors im Verhältnis zu dem elektrischen Signal um einen vorgegebenen Betrag verringert.

Es ist sowohl bei den bekannten Vorrichtungen als auch bei der erfindungsgemäßen Vorrichtung zum Detektieren von Herzereignissen nicht auszuschließen, daS Störsignale den jeweils aktuell eingestellten Schwellenwert überschreiten und fehlerhaft als Herzereignisse detektiert werden. Ein Beispiel dafür ist, daß bei einem intrakardialen Elektrogramm außer dem QRS- Komplex auch die T- Welle den Schwellenwert überschreitet, so daS in beiden Fällen jeweils ein Herzereignis detektiert wird. Um die Gefahr solcher Fehldetektionen zu verringern, ist entsprechend einer Weiterbildung der erfindungsgemäßen Vorrichtung vorgesehen, daß die Steuereinrichtung Mittel zum Bestimmen der Varianz der zur Bildung des Mittelwerts herangezogenen Signalhöhen aufweist und daß der Schwellenwert des Detektors um einen vorgegebenen Betrag erhöht wird, wenn die Varianz einen vorgegebenen Wert überschreitet. Normalerweise variiert nämlich die Signalhöhe von aufeinanderfolgenden normalen Herzereignissen nur gering, so daß eine höhere Varianz auf die Detektion von gegenüber den Herzereignissen unterschiedlichen Signalen zurückzuführen ist.

Da die Gefahr von Fehldetektionen umso größer ist, je höher die Detektionsempfindlichkeit beziehungsweise je niedriger der Schwellenwert eingestellt ist, und um zu verhindern, daß der Schwellenwert aufgrund der Fehldetektionen auf dem niedrigen Niveau hängen bleibt, wird die Bestimmung der Varianz vorzugsweise dann ausgelöst, wenn der aktuelle Schwellenwert des Schwellenwertdetektors unterhalb eines Mindestwerts eingestellt ist.

Zur Erläuterung der Erfindung wird im folgenden auf die Figuren der Zeichnung Bezug genommen; im einzelnen zeigen
- FIG 1: ein Beispiel für einen die erfindungsgemäße Vorrichtung beinhaltenden Herzschrittmacher,
- FIG 2: ein Blockschaltbild der erfindungsgemäßen Vorrichtung,
- FIG 3: ein Beispiel für die Einstellung der Detektionsempfindlichkeit der in Fig. 2 gezeigten Vorrichtung im Verhältnis zu dem zu erfassenden elektrischen Signal und
- FIG 4: ein Beispiel für die Änderung der Detektionsempfindlichkeit bei einer hohen Varianz der Detektionsmarginale.

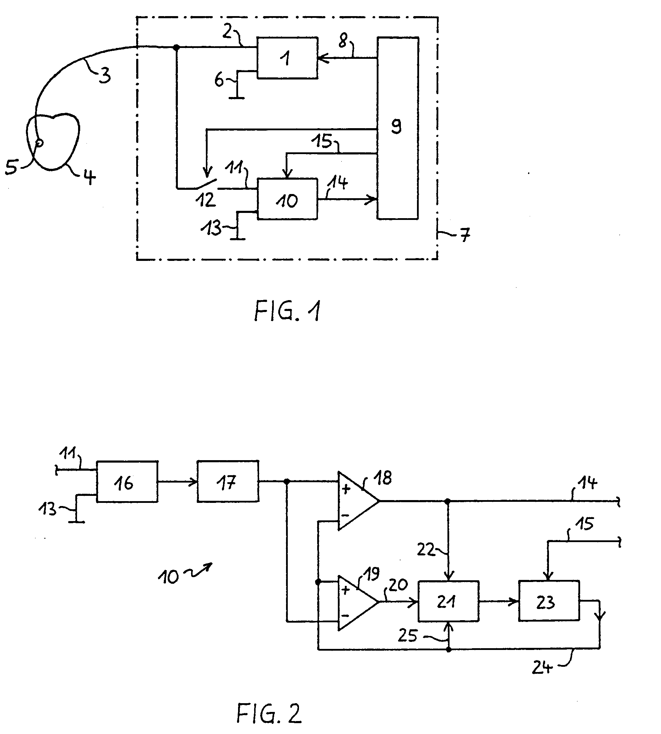

Figur 1 zeigt das Blockschaltbild eines Herzschrittmachers mit einem Stimulationsimpulsgenerator 1, der an einem ersten Ausgangsanschluß 2 über eine Elektrodenleitung 3 mit einer im Herzen 4 angeordneten Stimulationselektrode 5 verbunden ist. Der zweite Ausgangsanschluß 6 des Stimulationsimpulsgenerators 1 ist mit einem Gehäuse 7 des Herzschrittmachers verbunden, das als Gegenelektrode zur Stimulationselektrode 5 dient. Der Stimulationsimpulsgenerator 1 ist über eine Steuerleitung 8 mit einer Herzschrittmachersteuerung 9 verbunden, die über die Steuerleitung 8 die Abgabe von Stimulationsimpulsen durch den Stimulationsimpulsgenerator 1 veranlaßt. Eine Vorrichtung 10 zum Detektieren von spontanen Herzereignissen ist an einem ersten Eingangsanschluß 11 über einen von der Herzschrittmachersteuerung 9 steuerbaren Schalter 12 mit der Elektrode 5 verbunden und mit einem zweiten Eingangsanschluß 13 an dem Gehäuse 7 des Herzschrittmachers angeschlossen. Die Vorrichtung 10 ist über einen die Detektion eines Herzereignisses anzeigenden Ausgang 14 mit der Herzschrittmachersteuerung 9 verbunden und wird von dieser über eine weitere Steuerleitung 15 gesteuert.

Jedesmal nach Abgabe eines Stimulationsimpulses durch den Stimulationsimpulsgenerator 1 oder nach Detektion eines spontanen Herzereignisses durch die Vorrichtung 10 wird innerhalb der Herzschrittmachersteuerung 9 ein Basiszeitintervall gestartet. Zugleich wird nach Ablauf einer Refraktärzeit der steuerbare Schalter 12 durch die Herzschrittmachersteuerung 9 geschlossen, um das Auftreten eines natürlichen Herzereignisses durch die Vorrichtung 10 detektieren zu können. Wenn ein solches Herzereignis vor Ablauf des Basiszeitintervalls detektiert wird, wird das Basiszeitintervall erneut gestartet, ohne daß dabei ein Stimulationsimpuls erzeugt wird. Läuft dagegen das Basiszeitintervall ab, ohne daß ein natürliches Herzereignis detektiert wird, so wird ein Stimulationsimpuls an das Herz 4 abgegeben und das Basiszeitintervall erneut gestartet.

Figur 2 zeigt das Blockschaltbild eines Ausführungsbeispiels für die Vorrichtung 10 zur Detektion von Herzereignissen. Das zwischen den Eingangsanschlüssen 11 und 13 erfaßte elektrische Signal des intrakardialen Elektrogramms wird zunächst einer Filter- und Verstärkerstufe 16 zugeführt, in der aus dem elektrischen Signal die für die zu detektierenden Herzereignisse charakteristischen Freguenzanteile herausgefiltert werden. Ferner wird das Signal einer Signalvorbehandlung unterzogen, in der für die zu detektierenden Herzereignisse typische Signaleigenschaften wie z.B. eine vorgegebene Mindeststeilheit der Signalflanken oder eine bestimmte Signalbreite in eine Variation der Signalhöhe umgesetzt werden. Ein Beispiel hierfür kann die Differenzierung oder Integrierung des elektrischen Signals sein. Das so vorbehandelte elektrische Signal gelangt von der Filter- und Verstärkerstufe 16 auf einen Spitzenwertdetektor 17, in dem die Signalhöhe des elektrischen Signals ermittelt wird. Der Wert für die Signalhöhe wird dem nichtinvertierenden Eingang (+) eines Schwellenwertdetektors 18 zugeführt, dessen invertierender Eingang (-) mit einem einstellbaren Schwellenwert beaufschlagt ist. Wenn der Wert für die Signalhöhe den Schwellenwert überschreitet, gibt der Schwellenwertdetektor 16 über den Ausgang 14 der Vorrichtung 10 ein die Detektion eines Herzereignisses anzeigendes Ausgangssignal an die Herzschrittmachersteuerung 9 ab.

Zur Einstellung des Schwellenwerts wird dieser zusammen mit dem Wert für die Signalhöhe den beiden Eingängen einer Subtrahiereinrichtung 19 zugeführt, die an ihrem Ausgang 20 ein der Detektionsmarginale, also dem Abstand zwischen dem Schwellenwert und dem Wert der Signalhöhe entsprechendes Signal erzeugt. Die Detektionsmarginale, welche auch ein Maß für die Signalhöhe in bezug auf den Schwellenwert darstellt, wird einem Mittelwertbildner 21 zugeführt, der über einen ersten Steuereingang 22 mit dem Ausgang 14 des Schwellenwertdetektors 18 bzw. der Vorrichtung 10 verbunden ist und den Wert der Detektionsmarginale am Ausgang der Subtrahiereinrichtung 19 nur dann annimmt, wenn von dem Schwellenwertdetektor 18 das Vorliegen eines Herzereignisses detektiert worden ist. In dem Mittelwertbildner 21 wird aus dem neu eingelesenen Wert der Detektionsmarginale und einer vorgegebenen Anzahl von jeweils zuletzt eingelesenen Werten ein Mittelwert gebildet. Die Anzahl der zur Mittelwertbildung herangezogenen Werte kann beispielsweise 18 betragen, was bei einer normalen Herztätigkeit einer Mittlung über eine Dauer von 18 Herzschlägen oder ungefähr 3 Atemzügen entspricht. Dementsprechend enthält der Mittelwertbildner 21 einen hier nicht gezeigten Mittelwertspeicher mit einer Anzahl von 18 Speicherplätzen, wobei bei jedem detektierten Herzereignis der Wert der dabei ermittelten Detektionsmarginale anstelle des jeweils ältesten abgespeicherten Werts in den Speicher eingelesen wird.

Der in dem Mittelwertbildner 21 erzeugte Mittelwert wird in einer nachgeordneten Steuereinrichtung 23 zur Einstellung des dem Schwellenwertdetektor 18 und der Subtrahiereinrichtung 19 über einen Ausgang 24 der Steuereinrichtung 23 zugeführten Schwellenwerts herangezogen. Dabei erfolgt jede Änderung des Schwellenwerts mit einer Schalthysterese, indem der Schwellenwert erhöht wird, wenn der Mittelwert einen oberen Grenzwert der Schalthysterese überschreitet, und indem der Schwellenwert verringert wird, wenn der Mittelwert einen unteren Grenzwert der Schalthysterese unterschreitet. Der jeweilige Betrag der Änderung des Schwellenwerts ist so gewählt, daß der neue Schwellenwert in einem vorgegebenen Verhältnis zur Detektionsmarginale steht. Bei einem gewählten Verhältnis von 1 : 1 ist also die Signalhöhe doppelt so groß wie der Schwellenwert. Der Ausgang 24 der Steuereinrichtung 23 ist zusätzlich mit einem Rücksetzeingang 25 des Mittelwertbildners 21 verbunden. Über diesen Rücksetzeingang 25 werden nach jeder Änderung des Schwellenwerts die vor der Änderung in den Mittelwertbildner 21 eingelesenen Werte für die Detektionsmarginale zurückgesetzt, um bei der nachfolgenden Mittelwertbildung zu verhindern, daß die Werte für die auf dem neuen Schwellenwert beruhenden Detektionsmarginalen mit den Werten von den auf dem alten Schwellenwert beruhenden Detektionsmarginalen vermischt werden.

In der Steuereinrichtung 23 ist ferner eine Steuerfunktion implementiert, die dann, wenn der Schwellenwert unterhalb eines Mindestwerts eingestellt ist, einen Berechnungsalgorithmus zum Bestimmen der Varianz der dem Mittelwertbildner 21 zugeführten, den Schwellenwert übersteigenden Signalanteile aufweist; wenn die Varianz des Mittelwerts dabei einen vorgegebenen Wert überschreitet, wird der Schwellenwert um einen vorgegebenen Betrag erhöht.

Eine weitere Steuerfunktion der Steuereinrichtung 23 besteht darin, daß nach einer auf eine Detektion eines spontanten Herzereignisses erstmalig folgenden Abgabe eines Stimulationsimpulses, welche über die Steuerleitung 15 der Steuereinrichtung 23 gemeldet wird, der Schwellenwert um einen vorgegebenen Betrag verringert wird.

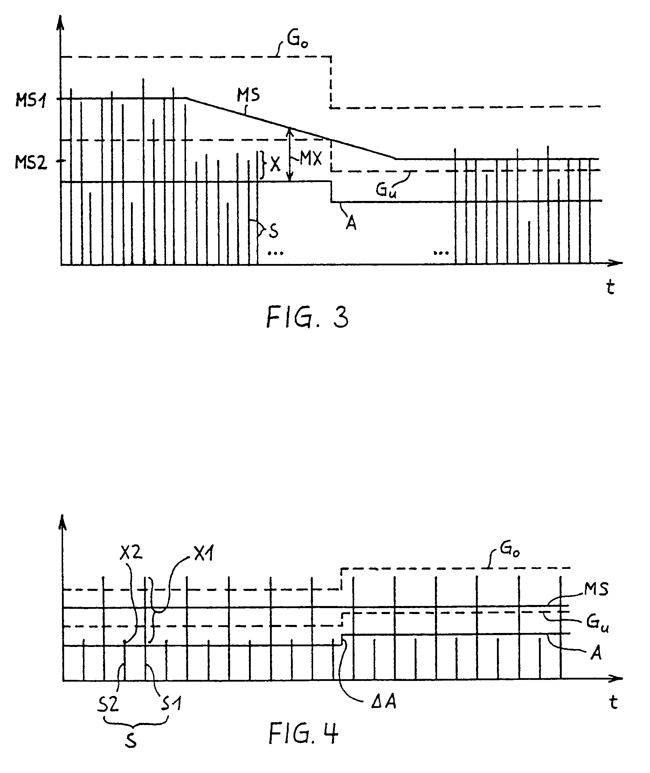

In Figur 3 ist in einem Diagramm ein Beispiel für die Änderung des Schwellenwerts A in Abhängigkeit von dem Mittelwert MX der Detektionsmarginale X zwischen dem elektrischen Signal S und dem Schwellenwert A dargestellt. Aus Gründen der einfacheren Darstellbarkeit ist das elektrische Signal S in Form von zeitdiskreten Abtastwerten unterschiedlicher Signalhöhe dargestellt; es kann sich bei dem Signal S aber auch um ein zeitkontinuierliches Signal handeln. Der über ein Zeitintervall von 20 aufeinanderfolgenden Abtastwerten gebildete Mittelwerte des elektrischen Signals S ist mit MS bezeichnet. Wie Figur 3 zeigt, schwanken anfangs die Signalhöhen des elektrischen Signals S um einen ersten Mittelwert MS1. Eine Detektion von Herzereignissen erfolgt jedesmal dann, wenn die Signalhöhe des elektrischen Signals S den vorgegebenen Schwellenwert A übersteigt. Der dabei den Schwellenwert A übersteigende Anteil der Signalhöhe bildet die Detektionsmarginale X. Der Mittelwert der Detektionsmarginalen x über eine vorbestimmte Anzahl von zurückliegenden Detektionsereignissen ist mit MX bezeichnet. Signalwerte des elektrischen Signals S, die den Schwellenwert A nicht überschreiten, tragen nicht zur Mittelwertbildung bei.

Solange der Verlauf des Mittelwerts MX der Detektionsmarginale zwischen dem unteren Grenzwert Gᵤ und dem oberen Grenzwert Gₒ einer Schalthysterese verläuft, wird der Schwellenwert A nicht verändert. Im Falle des in Figur 3 gezeigten Beispiels nehmen die Signalhöhen des elektrischen Signals S plötzlich ab und stabilisieren sich auf einen neuen Wert um MS2. Der Verlauf des Mittelwerts MX der Detektionsmarginale folgt der Veränderung der Signalhöhen mit einer durch die Mittelwertbildung bedingten Verzögerung. Da die Mittelwertbildung aber nur über relativ kurzes Zeitintervall erfolgt, ist die Verzögerung entsprechend gering. Sobald dabei der Mittelwert MX den unteren Grenzwert Gᵤ unterschreitet, wird der Schwellenwert A um einen bestimmten Betrag verringert, so daß sich dadurch der Mittelwert MX der Detektionsmarginale in bezug auf den neuen Schwellenwert A wieder auf ein Verhältnis von 1 : 1 einstellt. Mit der Änderung des Schwellenwerts A werden auch die Grenzwerte Gᵤ und Gₒ der Schalthysterese geändert, wobei allerdings das Verhältnis der Grenzwerte Gᵤ und Gₒ zu dem Schwellenwert A unverändert bleibt. Für den in Figur 3 nicht gezeigten Fall, daß der Verlauf des Mittelwerts MX der Detektionsmarginale den oberen Grenzwert Gₒ übersteigt, wird der Schwellenwert A um einen vorgegebenen Betrag erhöht, so daß dadurch wieder eine ausreichend hohe mittlere Detektionsmarginale MX erreicht wird.

Figur 4 zeigt ein Beispiel für die Regelung des Schwellenwerts A bei einer hohen Varianz der Signalhöhe des elektrischen Signals S. Dabei stellen die mit S1 bezeichneten Abtastwerte des elektrischen Signals S die zu detektierenden Herzereignisse, wie z.B. QRS-Komplex dar, während die mit S2 bezeichneten Abtastwerte Störsignale, wie z.B. die T-Wellen des Elektrokardiogramms darstellen. Der sich dabei ergebende Mittelwert MS des elektrischen Signals S führt durch die automatische Schwellenwertanpassung dazu, daß außer den Signalhöhen S1 der Herzereignisse auch die Signalhöhen S2 der Störsignale S2 den Schwellenwert A überschreiten und als Herzereignisse detektiert werden. Die hohe Varianz der den Schwellenwert A übersteigenden Signalanteile X1, X2 weist jedoch auf Fehldetektionen hin. Bei der Mittelwertbildung wird daher die Varianz der zur Mittelwertbildung herangezogenen Detektionsmarginalwerte X1, X2 berechnet. Wenn die Varianz, wie im Beispiel der Figur 4 dabei einen vorgegebenen Wert überschreitet, wird der Schwellenwert A um einen vorgegebenen Betrag Δ A erhöht, so daß die Störsignale S2 nicht mehr fälschlicherweise als Herzereignisse detektiert werden.

Wenn auch in Figur 2 Schaltungsblöcke der erfindungsgemäßen Vorrichtung zur Detektion von Herzereignissen dargestellt sind, so sind diese Schaltungsblöcke vorzugsweise als Funktionsblöcke zu verstehen. Insbesondere können die beschriebenen Funktionen als ein Programmablauf in der Vorrichtung 10 implementiert sein. Da die Mittelwertbildung, wie beschrieben, nur über ein relativ kurzes Zeitintervall bzw. nur wenige Abtastwerte des elektrischen Signals erfolgt, ist der dazu erforderliche Rechenaufwand entsprechend gering.

### Bezugszeichenliste

- 1: Stimulationsimpulsgenerator
- 2: erster Ausgangsanschluß von 1
- 3: Elektrodenleitung
- 4: Herz
- 5: Stimulationselektrode
- 6: zweiter Ausgangsanschluß von 1
- 7: Gehäuse
- 8: Steuerleitung
- 9: Herzschrittmachersteuerung
- 10: Vorrichtung zum Detektieren von Herzereignissen
- 11: erster Eingangsanschluß von 10
- 12: steuerbarer Schalter
- 13: zweiter Eingangsanschluß von 10
- 14: Ausgang von 10
- 15: weitere Steuerleitung
- 16: Filter- und Verstärkerstufe
- 17: Spitzenwertdetektor
- 18: Schwellenwertdetektor
- 19: Subtrahiereinrichtung
- 20: Ausgang von 19
- 21: Mittelwertbildner
- 22: erster Steuereingang von 21
- 23: Steuereinrichtung
- 24: Ausgang von 23
- 25: Rücksetzeingang von 21
- A: Schwellenwert
- A: Änderungsbetrag von A
- Gₒ: oberer Grenzwert einer Schalthysterese
- Gᵤ: unterer Grenzwert der Schalthysterese
- MS: Verlauf des Mittelwerts von S
- MS1, MS2: mittlere Werte von S
- MX: Mittelwert von X
- S,S1, S2: elektrisches Signal
- X,X1, X2: den Schwellenwert A übersteigender Anteil von S (Detektionsmarginale)

## Patentansprüche

1. Vorrichtung zum Detektieren von Herzereignissen mit einem Signalaufnehmer (5) zum Erfassen eines von den Herzereignissen abhängigen elektrischen Signals (S), einem dem Signalaufnehmer (5) nachgeordneten Schwellenwertdetektor (18) zum Erzeugen eines das vorliegen eines Herzereignisses anzeigenden Detektionssignals, wenn das elektrische Signal (S) einen vorgegebenen Schwellenwert (A) überschreitet, mit einer Signalauswerteeinrichtung (19) zur Bestimmung der aktuellen Signalhöhe des den Schwellenwert (A) übersteigenden elektrischen Signals (S) in bezug auf den Schwellenwert (A), mit einem der Signalauswerteeinrichtung (19) nachgeordneten Mittelwertbildner (21) zum Bilden eines Mittelwerte (MX) aus den während eines vorgegebenen Zeitintervalls bestimmten schwellenwertbezogenen Signalhöhen (X) und mit einer Steuereinrichtung (23) zum Einstellen des Schwellenwerts (A) im Verhältnis zu dem erfaßten elektrischen Signal (S) in Abhängigkeit von dem gebildeten Mittelwert (MX), wobei der Schwellenwert (A) im Verhältnis zu dem elektrischen Signal (S) bei zunehmendem Mittelwert (MX) erhöht und bei abnehmendem Mittelwert (MX) verringert wird, **dadurch gekennzeichnet**, daß das Zeitintervall zur Mittelwertbildung weniger als eine Minute betragt und einer Dauer von einigen Atemzügen entspricht und daß die Steuereinrichtung (23) Mittel zum Erzeugen einer Schalthysterese mit einem unteren Grenzwert (Gᵤ) und einem oberen Grenzwert (Gₒ) aufweist, wobei eine Neueinstellung des Schwellenwerts (A) jeweils nur dann erfolgt, wenn der Mittelwert (MX) entweder dem unteren Grenzwert (Gᵤ) unterschreitet oder den oberen Grenzwert (Gₒ) überschreitet.

2. Vorrichtung nach Anspruch 1, **dadurch gekennzeichnet**, daß nach jeder Neueinstellung des Schwellenwerts (A) alle zur Bildung des Mittelwerts (MX) herangezogenen Signalhöhen auf den neuen Schwellenwert (A) bezogen sind.

3. Vorrichtung nach Anspruch 1 oder 2, **dadurch gekennzeichnet**, daß die Länge des Zeitintervalls durch eine vorbestimmte Anzahl von vorzugsweise 10 bis 30 aufeinanderfolgend detektierten Herzereignissen vorgegeben ist.

4. Vorrichtung nach einem der vorangehenden Ansprüche, **dadurch gekennzeichnet**, daß die Vorrichtung (10) Bestandteil eines Herzschrittmachers ist, bei dem die Abgabe eines Stimulationsimpulses dann erfolgt, wenn nicht innerhalb eines auf eine vorangegangene Stimulation oder Detektion eines spontanen Herzereignisses folgenden Basiszeitintervalls eine spontanes Herzereignis durch die Vorrichtung (10) detektiert wird, und daß nach einer auf eine Detektion eines spontanen Herzereignisses erstmalig folgenden Abgabe eines Stimulationsimpulses der Schwellenwert (A) des Schwellenwertdetektors (18) im Verhältnis zu dem elektrischen Signal (S) um einen vorgegebenen Betrag verringert wird.

5. Vorrichtung nach einem der vorangehenden Ansprüche, **dadurch gekennzeichnet**, daß die Steuereinrichtung (23) Mittel zum Bestimmen der Varianz der zur Bildung des Mittelwerts (MX) herangezogenen Signalhöhen aufweist und daß der Schwellenwert (A) des Schwellenwertdetektors (18) um einen vorgegebenen Betrag erhöht wird, wenn die Varianz einen vorgegebenen Wert überschreitet.

6. Vorrichtung nach Anspruch 5, **dadurch gekennzeichnet**, daß die Bestimmung der Varianz dann ausgelöst wird, wenn der aktuelle Schwellenwert (A) des Schwellenwertdetektors (18) unterhalb eines Mindestwerts eingestellt ist.

## Claims

1. Device for detecting heart events, having a signal sensor (5) for detecting an electrical signal (S) which is dependent on the heart events, a threshold value detector (18) arranged downstream of the signal sensor (5) for generating a detection signal, which indicates the presence of a heart event, if the electrical signal (S) exceeds a specified threshold value (A), having a signal evaluating device (19) for determining the current signal level of the electrical signal (S) which exceeds the threshold value (A) in relation to the threshold value (A), having an averaging unit (21) arranged downstream of the signal evaluating device (19) for forming an average value (MX) from the threshold-related signal levels (X) which are determined during a specified time interval, and having a control device (23) for adjusting the threshold value (A) in relation to the electrical signal (S) which is detected, as a function of the average value (MX) which is formed, with the threshold value (A) being increased in relation to the electrical signal (S) in the case of an increasing average value (MX) and reduced in the case of a decreasing average value (MX), characterised in that the time interval for forming the average value amounts to less than one minute and corresponds to a duration of a few breaths, and in that the control device (23) has means for generating a switching hysteresis having a lower limiting value (Gᵤ) and an upper limiting value (Gₒ), with a readjustment of the threshold value (A) taking place in each case only if the average value (MX) either falls below the lower limiting value (Gᵤ) or exceeds the upper limiting value (Gₒ).

2. Device according to claim 1, characterised in that after each readjustment of the threshold value (A), all signal levels used to form the average value (MX) are applied to the new threshold value (A).

3. Device according to claim 1 or 2, characterised in that the length of the time interval is specified by a predetermined number of preferably 10 to 30 successively detected heart events.

4. Device according to one of the preceding claims, characterised in that the device (10) is part of a heart pacemaker, in which the delivery of a stimulation pulse takes place if a spontaneous heart event is not detected by the device (10) within a base time interval which follows a previous stimulation or detection of a spontaneous heart event, and in that after a delivery of a stimulation pulse that follows for the first time a detection of a spontaneous heart event, the threshold value (A) of the threshold value detector (18) is reduced in relation to the electrical signal (S) by a specified amount.

5. Device according to one of the preceding claims, characterised in that the control device (23) has means for determining the variance of the signal levels used to form the average value (MX), and in that the threshold value (A) of the threshold value detector (18) is increased by a specified amount if the variance exceeds a specified value.

6. Device according to claim 5, characterised in that the determining of the variance is triggered if the current threshold value (A) of the threshold value detector (18) is adjusted below a minimum value.

## Revendications

1. Dispositif pour détecter des événements cardiaques comportant un capteur (5) de signal pour détecter un signal (S) électrique dépendant des événements cardiaques, un détecteur (18) à valeur de seuil, monté en aval du capteur (5) de signal pour produire un signal de détection indiquant la présence d'un événement cardiaque, si le signal (S) électrique est supérieur à une valeur (A) de seuil prescrite, un dispositif (19) d'exploitation de signal pour déterminer le niveau du moment par rapport à la valeur (A) de seuil du signal (S) électrique supérieur à la valeur (A) de seuil, un dispositif (21) de formation de valeur moyenne monté en aval du dispositif (19) d'exploitation de signal pour former une valeur (MX) moyenne des niveaux (X) de signal par rapport à la valeur de seuil déterminés pendant un intervalle de temps prescrit, et un dispositif (23) de commande pour régler la valeur (A) de seuil par rapport au signal (S) électrique détecté en fonction de la valeur (MX) moyenne formée, la valeur (A) de seuil étant augmentée par rapport au signal (S) électrique lorsque la valeur moyenne (MX) croît et étant diminuée lorsque la valeur moyenne (MX) décroît, caractérisé en ce que l'intervalle de temps pour la formation de la valeur moyenne est inférieur à une minute et correspond à une durée de quelques inspirations et en ce que le dispositif (23) de commande comporte des moyens servant à produire une hystérésis de commande ayant une valeur (Gᵤ) limite inférieure et une valeur (Gₒ) limite supérieure, un nouveau réglage de la valeur (A) seuil n'ayant lieu que si la valeur (MX) moyenne soit passe au-dessous de la valeur limite (Gᵤ) inférieure soit dépasse la valeur (Gₒ) limite supérieure.

2. Dispositif suivant la revendication 1, caractérisé en ce que, après chaque nouveau réglage de la valeur (A) de seuil, tous les niveaux de signal utilisés pour la formation de la valeur (MX) moyenne sont rapportés à la nouvelle valeur (A) de seuil.

3. Dispositif suivant la revendication 1 ou 2, caractérisé en ce que la longueur de l'intervalle de temps est prescrite par un nombre prédéterminé de préférence de 10 à 30 événements cardiaques détectés successivement.

4. Dispositif suivant l'une des revendications précédentes, caractérisé en ce que le dispositif (10) fait partie d'un stimulateur cardiaque, dans lequel l'envoi d'une impulsion de stimulation a lieu si le dispositif (10) ne détecte pas un événement cardiaque spontané à l'intérieur d'un intervalle de temps de base suivant une stimulation précédente ou une détection précédente d'un événement cardiaque spontané, et en ce que, après l'envoi d'une impulsion de stimulation suivant pour la première fois une détection d'un événement cardiaque spontané, la valeur (A) de seuil du détecteur (18) à valeur de seuil est diminuée d'une valeur prescrite par rapport au signal (S) électrique.

5. Dispositif suivant l'une des revendications précédentes, caractérisé en ce que le dispositif (23) de commande comporte des moyens servant à déterminer la variance des niveaux de signal utilisés pour la formation de la valeur (MX) moyenne et en ce la valeur (A) de seuil du détecteur (18) à valeur de seuil est augmentée d'une valeur prescrite lorsque la variance est supérieure à une valeur prescrite.

6. Dispositif suivant la revendication 5, caractérisé en ce que la détermination de la variance est déclenchée si la valeur (A) de seuil du moment du détecteur (18) à valeur de seuil est réglée au-dessous d'une valeur minimale.
